Europäisches Patentamt

European Patent Office

Office européen des brevets

(11) Publication number: **0 211 835**
**B1**

## EUROPEAN PATENT SPECIFICATION

(45) Date of publication of patent specification: **03.01.90**

(51) Int. Cl.⁵: **A 61 K 37/02**

(21) Application number: **85901824.4**

(22) Date of filing: **25.03.85**

(86) International application number:
**PCT/US85/00501**

(87) International publication number:
**WO 85/04328 10.10.85 Gazette 85/22**

(54) **PHARMACEUTICAL COMPOSITIONS OF MICROBIALLY PRODUCED INTERLEUKIN-2.**

<table>
<tr><td>

(30) Priority: **28.03.84 US 594350**

(43) Date of publication of application:
**04.03.87 Bulletin 87/10**

(45) Publication of the grant of the patent:
**03.01.90 Bulletin 90/01**

(84) Designated Contracting States:
**AT BE CH DE FR GB LI NL SE**

(56) References cited:
**EP-A- 91 539**
**EP-A- 109 748**

**NATURE, vol. 302, no. 5906, March 1983,
Chesham, Bucks (GB); T.TANIGUCHI et al.:
"Structure and expression of a cloned cDNA for
human interleukin-2", pp. 305-310**

</td><td>

(73) Proprietor: **CETUS CORPORATION**
**1400 Fifty-Third Street**
**Emeryville California 94608 (US)**

(72) Inventor: **FERNANDES, Peter**
**216 Happy Hollow Court**
**Lafayette, CA 94596 (US)**
Inventor: **TAFORO, Terrance**
**3154 Wisconsin**
**Oakland, CA 94602 (US)**

(74) Representative: **Bizley, Richard Edward et al**
**BOULT, WADE & TENNANT 27 Furnival Street**
**London EC4A 1PQ (GB)**

</td></tr>
</table>

Courier Press, Leamington Spa, England.

## Description

This invention is in the field of pharmaceuticals. More particularly, it relates to pharmaceutical formulations of microbially produced interleukin-2.

Interleukin-2, a lymphokine which is produced by normal peripheral blood lymphocytes and induces proliferation of antigen or mitogen stimulated T cells after exposure to plant lectins, antigens, or other stimuli, was first described by Morgan, D. A., et al., *Science* 1976 *193*:1007—1008. Then called T cell growth factor because of its ability to induce proliferation of stimulated T lymphocytes, it is now recognized that in addition to its growth factor properties it modulates a variety of functions of immune system cells *in vitro* and *in vivo* and has been renamed interleukin-2 (IL-2). IL-2 is one of several lymphocyte-produced messenger-regulatory molecules that mediate immunocyte interactions and functions.

IL-2 was initially made by cultivating human peripheral blood lymphocytes (PBL) or other IL-2-producing cell lines. See, for instance, US—A—4,401,756. Recombinant DNA technology has provided an alternative to PBLs and cell lines for producing IL-2. Taniguchi, T., et al., *Nature* (1983) *302*:305—310 and Devos, R., *Nucleic Acids Research*, (1983) *11*:4307—4323 have reported cloning the human IL-2 gene and expressing it in microorganisms.

BE—A 898,016, granted 14 November 1983 describes muteins of IL-2 in which the cysteine normally occurring at position 125 of the wild-type or native molecule has been deleted or replaced with a neutral amino acid, such as serine. These muteins possess IL-2 biological activity. The Belgian patent states that the recombinant muteins may be formulated and administered as with native IL-2 by combining them with aqueous vehicles and injecting them intravenously, subcutaneously, or the like.

One aspect of the present invention is a recombinant IL-2 composition suitable for reconstituting in a pharmaceutically acceptable aqueous vehicle for parenteral administration to a patient to provide IL-2 therapy comprising a sterile lyophilized mixture of:

(a) a therapeutically effective amount of selectively oxidized microbially produced recombinant IL-2 that is substantially free of non-IL-2 protein;

(b) a pharmaceutically acceptable water soluble carrier that does not affect the stability of the oxidized microbially produced IL-2 adversely; and

(c) a sufficient amount of surface active agent such as alkali metal alkyl sulfates, e.g., sodium dodecyl sulfate (SDS), alkali metal sarcosinates or sodium deoxycholate to ensure the water solubility of the oxidized, microbially produced recombinant IL-2.

Preferably, the recombinant IL-2 has been selectively oxidized such that the cysteines at positions 69 and 105 form a disulfide bond to render the molecule biologically active.

Another aspect of this invention is a pharmaceutical composition for providing therapy to a patient comprising a sterile solution of:

(a) the above described mixture dissolved in

(b) a pharmaceutically acceptable aqueous parenteral vehicle, said solution containing in the range of 0.01 mg to 2 mg of the microbially produced recombinant IL-2 per ml.

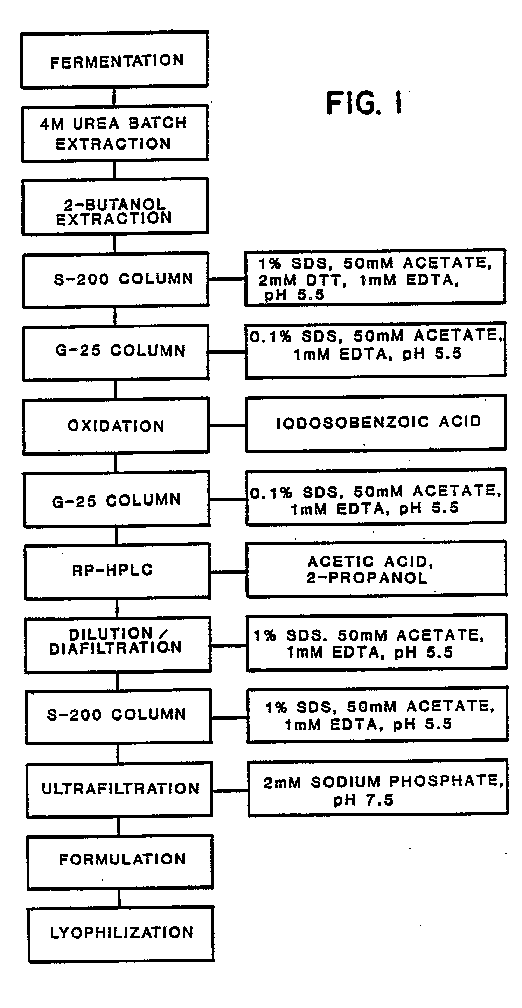

Figure 1 is a flow diagram of a preferred procedure for processing and purifying microbially produced recombinant IL-2.

As used herein the term "IL-2" denotes an unglycosylated protein that is (a) produced by a microorganism that has been transformed with a human interleukin-2 DNA sequence or a modification of the human interleukin-2 DNA sequence that encodes a protein having: (a) an amino acid sequence that is at least substantially identical to the amino acid sequence of native human interleukin-2 including the disulfide bond of the cystines at positions 58 and 105, and (b) has biological activity that is common to native human interleukin-2. Substantial identity of amino acid sequences means the sequences are identical or differ by one or more amino acid alterations (deletions, additions, substitutions) that do not cause an adverse functional dissimilarity between the synthetic protein and native human interleukin-2. Examples of such proteins are the recombinant IL-2s described in EP—A—91539 and EP—A—88195, the recombinant IL-2 muteins described in EP—A—109748 which is the equivalent to BE—A—898,016, and the recombinant IL-2s described in this application.

As used herein the term "transformed microorganism" denotes a microorganism that has been genetically engineered to produce a protein that possesses native human interleukin-2 activity. Examples of transformed microorganisms are described in said EP—A—88,195; 91,539 and 109,748. Bacteria are preferred microorganisms for producingt IL-2. A typical transformed microorganism useful in the present invention is *E. coli* K-12 strain MM294 transformed with plasmid pLW1 (deposited at the American Type Culture Collection on August 4, 1983 by Cetus Corporation under the provisions of the Budapest Treaty and having accession number 339,405). Synthetic recombinant IL-2 may also be made by suitably transformed yeast and mammalian cells. *E. coli* is particularly preferred host organism.

The transformed microorganisms are grown in a suitable growth medium, typically to an optical density (OD) of at least about 30 at 680 nm, and preferably between about 20 and 40 at 680 nm. The composition of the growth medium will depend upon the particular microorganism involved. The medium is an aqueous medium containing compounds that fulfill the nutritional requirements of the microorganism. Growth media will typically contain assimilable sources of carbon and nitrogen, energy sources, magnesium, potassium and sodium ions, and optionally amino acids and purine and pyrimidine bases. (See *Review of Medical Biology*, Lange Medical Publications, 14th Ed., pp 80—95 (1980).) In expression vectors involving the trp promoter, the tryptophane concentration in the medium is carefully controlled to become limiting at the time IL-2 expression is desired. Growth media for *E. coli* are well known in the art. A preferred growth method is described in U.S. Patent No. 4,499,188, granted 12 February 1985.

After the cells are harvested from the culture, they may be concentrated, if necessary, to about 20 to 150 mg/ml, preferably 80 to 100 mg/ml (OD 40 to 300, preferably 160 to 200 at 680 nm) by filtration, centrifugation, or other conventional methods.

Following concentration the cell membranes of the microorganisms are disrupted. The main purpose of disruption is to facilitate the following extraction and solubilization steps. Conventional cell disruption techniques such as homogenization, sonication, or pressure cycling may be used in this step of the process. Preferred methods are sonication or homogenization with a Manton-Gaulin homogenizer. The end point of the disruption step may be monitored by optical density, with the optical density of the suspension typically decreasing about 65% to 85%. In any event, the disruption should break substantially all of the cells so that substantially no intact cells are carried through to the solubilization step. Before the disruption, the pH of the liquid phase of the concentrate is adjusted, if necessary, to a level that facilitates removal of *E. coli* proteins in subsequent steps, while retaining recombinant IL-2 protein as an insoluble complex in the cellular debris. The pH may be so adjusted by adding suitable buffers. In most instances pHs in the range of about 8 to about 8.5 will be used.

The steps in the recovery process subsequent to the disruption step as shown in Fig. 1 are primarily designed to separate the IL-2 from *E. coli* proteins to a high level of purity (preferably at least about 95% and more preferably at least about 98%) in good yields while maintaining the IL-2 in a reduced state. Simultaneously, these purification processes, in combination, also reduce pyrogenic substances in the final product to a level believed to be acceptable for parenteral administration to patients.

After the cells have been disrupted the particulate matter may be separated from the liquid phase of the disruptate and resuspended in an aqueous medium buffered to the optimal pH for the extraction. The particulate matter may optionally be washed with buffer at this stage to remove any water soluble *E. coli* proteins therein. In any event, the protein concentration of the cell suspension subjected to the extraction will usually be in the range of about 5 to about 60 mg/ml, preferably 20 to 40 mg/ml.

The extraction of *E. coli* proteins from the particulate cellular material may be carried out concurrently with the disruption or sequentially following the disruption. It is preferably carried out as a step following the disruption. The extractant is an aqueous solution of a chaotropic agent (i.e., a mild protein denaturant that dissociates hydrogen bonds and affects the tertiary structure of proteins). The extractant selectively removes the bulk of the *E. coli* proteins from the cellular debris leaving at least a substantial portion of the recombinant IL-2 associated (contained in or bound to) with the cellular debris. The selectivity is facilitated by the hydrophobicity of the recombinant IL-2 and the fact that it is in a reduced, insoluble state at a pH near the isoelectric point of the protein. In addition, a substantial portion of the recombinant IL-2 may be present in *in vivo* as inclusion bodies of significant mass, as has been the case with other cloned proteins expressed at high levels in *E. coli*. Examples of extractants are urea and guanidinium hydrochloride (guanidinium hydrochloride should not be used when SDS is used as a solubilizing agent). Urea is preferred. The concentration of the chaotropic agent in the extraction mixture will depend upon the particular agent that is used and the amount of cellular material in the extraction mixture. In the case of urea, concentrations (final) between about 3.5 M and 4.5 M, preferably about 4 M, will be used in batch processes at 25°C. If the extraction is run on a continuous basis over longer time periods it may be desirable to use lower concentrations. Temperatures in the range of 20°C to 25°C will normally be used in extraction, with room temperature being used for convenience. Mixing will typically be used to enhance contact between the solution and particulate matter and thus decrease the time required to extract non-IL-2 proteins from the cellular debris. Kinetic analysis of the extraction process was performed on the supernatants using SDS-PAGE, and the extraction was found to be essentially complete by 15—30 minutes.

Following the extraction, the mixture is separated into solid and liquid phases. The recombinant IL-2 in the solid phase is then selectively solubilized by contacting the solid phase with a neutral, aqueous buffer containing a reducing agent and a solubilizing agent. Physiologically acceptable surface active agents (detergents) that have a suitable hydrophobic-hydrophilic balance to solubilize the hydrophobic recombinant IL-2 may be used. Alkali metal alkyl sulfates containing 10 to 14 carbon atoms and alkali metal alkyl sarcosinates are preferred solubilizing agents, with SDS and sarcosyl® being particularly preferred.

The amount of solubilizing agent used in the solubilization will depend upon the particular agent. When SDS or sarcosyl (registered Trade Mark) are used, the preferred ratio (w/w) of SDS (sarcosyl) to solid phase protein is about 0.5:1 to 1.4:1. The solubilizing medium also contains a sufficient amount of reducing agent to prevent the solubilized IL-2 from undergoing oxidation to any significant degree. Protein reducing agents such as dithiothreitol (DTT) and 2-mercaptoethanol may be used. The concentration of reducing agent such as DTT in the medium will usually range between about 5 to 20 mM. The solubilization will typically be carried out at temperatures in the range of 20°C to 25°C with mixing to facilitate contact between the solid phase and the solubilizing medium. Higher temperatures may solubilize unwanted *E. coli* proteins. The solubilization is considered complete when the sample has stood 15 minutes or the solution turns translucent. Insoluble material is separated after completing the solubilization.

After the IL-2 is solubilized the IL-2 may optionally be extracted from the aqueous solution under reducing conditions with 2-butanol or 2-methyl-2-butanol to remove additional *E. coli* proteins, notably including certain contaminants that have molecular weights very close to the IL-2. Conditions (e.g., ionic strengths in the range of 0.05 and 0.15) at which the aqueous solution and butanol are substantially immiscible are used. In carrying out the organic extraction the protein concentration of the aqueous solution is preferably adjusted, if necessary, to less than about 6 mg/ml, preferably about 0.5 to 4 mg/ml. Reducing conditions are maintained by carrying out the extraction in the presence of a reducing agent (e.g., DTT). The butanol will normally be added to the aqueous solution of solubilized IL-2 in volume ratios in the range of about 1:1 to about 3:1 (extractant:aqueous solution), preferably about 1:1. The extraction may be carried out in a batch or continuous operation. The temperature will normally be in the range of 20°C to 100°C and the pH will normally be about 4 to 9, preferably about 5 to 6. The time of contact between the solution and the butanol is not critical and relatively short times on the order of a few minutes may be used. After the extraction is complete, the aqueous phase and butanol phase are separated and the IL-2 is separated from the butanol phase. A preferred procedure for separating the IL-2 from the butanol phase is acid precipitation. This is done by adding the butanol phase to aqueous buffer, pH 7.5 until the organic phase is dissolved (approx. 2—3 vol buffer per vol of organic), and then lowering the pH to about 5.5 to 7.0, preferably 6.0 to 6.2, to cause the IL-2 to precipitate.

The next step in the process is to separate the recombinant IL-2 and any *E. coli* contaminants remaining after the extraction(s) and optimally from the solubilizing agent. Gel filtration chromatography, RP-HPLC, or a combination of gel filtration chromatography and RP-HPLC are used. The gel filtration chromatographic is preferably carried out in two stages that remove pyrogenic components and protein contaminants having molecular weights higher or lower than recombinant IL-2. (Recombinant IL-2 has a molecular weight of about 15.5K daltons.) Gels that are capable of fractionating the solution to permit separation of the IL-2 from these contaminants are commercially available. Sephacryl (registered Trade Mark) S—200 is a preferred gel for removing the higher molecular weight components and Sephadex (registered Trade Mark) G—25, G—75 or G—100 gels are preferred for removing the low molecular weight contaminants. The gel filtrations will typically be run in buffered solutions (pH 5.5 to 7.0) containing about 0.1% to 1.0% solubilizing agent and about 1 to 10 mM reducing agent. The column will be sized to permit suitable resolution of the desired components.

RP-HPLC is an alternative to gel filtration. Also, RP-HPLC is capable of removing molecules from the solution that have molecular weights close to recombinant IL-2 and cannot, therefore, be removed completely by gel filtration. In addition, contaminants such as bacterial endotoxin are also removed effectively by RP-HPLC. Therefore, RP-HPLC may also be used as a final purification step after gel filtration. Supports (stationary phases) that provide good resolution of proteins may also be used as a final purification step after gel filtration. Supports (stationary phases) that provide good resolution of proteins may be used in the RP-HPLC. C-4, C-8, or C-18 on 300 angstrom pore-size supports are examples of preferred supports. The separation is carried out at an acidic pH of less than about 2.3, usually 2.1 to 2.3 in order to keep the IL-2 in solution. In this regard, the pH of the solution from the solubilization (gel filtration) will preferably be adjusted to this range. The solution is loaded into the RP-HPLC column and is absorbed onto the stationary phase. A gradient solvent system comprising an organic acid such as acetic acid or trifluoracetic acid and organic solvent such as propanol or acetonitrile is used to elute the recombinant IL-2 from the column. Acetic acid-propanol, trifluoroacetic acid-propanol, and trifluoroacetic acid-acetonitrile are preferred solvent systems. Recombinant IL-2 elutes in the acetic acid-propanol system at about 40% propanol, in the trifluoroacetic acid-propanol system at about 50% propanol, and in the trifluoroacetic acid-acetonitrile system at about 62% acetonitrile. For convenience, the organic solvent content of the elutant will usually be increased rapidly to a level somewhat below the solvent concentration at which the recombinant IL-2 elutes followed by a slow gradient change in the range of about 0.1% to 1.0%/min.

As soon as the recombinant IL-2 is recovered from the chromatography step, it is lyophilized and resuspended in a neutral aqueous buffer containing the reducing agent (to keep the recombinant IL-2 in a reduced state) and the solubilizing agent (to keep it in solution). The recombinant IL-2 is stable in this form and may be stored for further treatment and formulation before being used.

According to the invention the recombinant IL-2 is selectively oxidized, under controlled conditions, after it has been separated by gel filtration; the oxidized product is purified by RP-HPLC or gel filtration followed by RP-HPLC. This results in efficient removal of contaminants surviving the gel filtration as well as unwanted oxidation products. A preferred oxidation procedure is to selectively oxidize a fully reduced microbially produced synthetic recombinant IL-2 protein having an amino acid sequence substantially identical to the recombinant IL-2 protein which sequence includes cysteines which in the useful protein are linked intramolecularly at positions 58 and 105 to form a cystine in a controlled manner so that the cysteines are oxidized selectively to form the cystine at positions 58 and 105. The efficiency of the controlled and selective oxidation is improved if a recombinant IL-2 mutein is used such as described and claimed in BE—A—898 016. In such case the cysteine at position 125 is deleted or replaced with a neutral amino acid thus preventing incorrect intramolecular bonds and/or intermolecular bonds with the cysteine at position 125 during oxidation which may also form dimers or polymers of IL-2. In this process the fully reduced microbially produced synthetic recombinant IL-2 protein is preferably reacted with o-iodosobenzoate, which oxidizes cysteines selectively in an aqueous medium, at a pH at least about one-half pH unit below the $pK_a$ of said cysteines, wherein the concentration of synthetic protein in the reaction mixture is less than about 5 mg/ml and the mol ratio of o-iodosobenzoate to protein is at least stoichiometric, with the proviso that the o-iodosobenzoate is in excess in the terminal portion of the reaction. This selective oxidation produces a biologically active molecule. RP-HPLC purification of the selectively oxidized product may be carried out under the conditions described above in the absence of a reducing agent and presence of a detergent at a concentration equal to or less than those used in the above described gel filtration.

The purity of the recombinant IL-2 after the chromatography step(s) is at least about 95% and usually at least about 98%. This highly pure material contains less than about 5 ng endotoxin, usually less than about 0.01 ng endotoxin per 100,000 Units IL-2 activity.

The formulation of recombinant IL-2 in accordance with this invention may be carried out as a separate operation using purified, selectively oxidized IL-2 or in an operation that is integrated with the purification of the selectively oxidized IL-2. In the latter case, the starting material for the formulation is a recombinant IL-2-containing product from a reverse phase high performance liquid chromatography (RP-HPLC) treatment of the selectively oxidized product, preferably one selectively oxidized by the RP-HPLC product (pool) will comprise a solution of recombinant IL-2 in a water-organic solvent mixture. The nature of the organic solvent will depend upon the solvent system used in RP-HPLC. Examples of systems that may be used are combinations of an organic acid such as acetic acid or trifluoroacetic acid and organic solvent such as propanol or acetonitrile.

The first step in formulating the recombinant IL-2 from such an RP-HPLC pool is to render the mixture aqueous by resuspending (diluting) the pool in an aqueous buffer containing a detergent, such as SDS or sarcosyl, that enhances the solubility of the recombinant IL-2 in water. Following this dilution the organic phase is removed from the recombinant IL-2 containing aqueous phase and the detergent concentration is reduced by diafiltration using an appropriate buffer. When SDS is used, the SDS is reduced to a level of about 100 to 250, preferably approximately 200, µg/mg IL-2. Following diafiltration, the IL-2 concentration is readjusted to a concentration in the range of about 0.01 to 2 mg/ml and the water soluble carrier is added to the desired level. The carrier will typically be added such that it is present in the solution at about 1 to 10% by weight, preferably about 5% by weight. The exact amount of carrier added is not critical. Conventional solid bulking agents that are used in pharmaceutical tablet formation may be used as the carrier. These materials are water soluble, do not react with the IL-2, and are themselves stable. They are also preferably non-sensitive (i.e., nonhygroscopic) to water. Examples of carriers that may be added are lactose, mannitol, and other reduced sugars such as sorbitol, starches and starch hydrolysates derived from wheat, corn, rice, and potato, microcrystalline celluloses, and albumin such as human serum albumin. Mannitol is preferred.

The carrier adds bulk to the formulation such that when unit dosage amounts of the solution are lyophilized in containers, such as sterile vials, the freeze-dried residue will be clearly discernible to the naked eye. In this regard the preferred carrier, mannitol, yields an aesthetically acceptable (white, crystalline) residue that is not sensitive to water. The nonsensitivity of mannitol to water may enhance the stability of the formulation.

After adding the carrier the unit dosage amounts (i.e., volumes that will provide 0.01 to 2 mg, preferably 0.2 to 0.3 mg, IL-2 per dose) of the solution are dispensed into containers, the containers are capped with a slotted stopper, and the contents are lyophilized using conventional freeze-drying conditions and apparatus.

The lyophilized, sterile product consists of a mixture of (1) recombinant IL-2, (2) carrier (mannitol), (3) detergent (SDS), and (4) a small amount of buffer that will provide a physiological pH when the mixture is reconstituted. The recombinant IL-2 will typically constitute 0.015% to 3.85% by weight of the mixture, e.g. 0.02% to 3.85% by weight of the mixture, more preferably 0.4% to 0.6% of the mixture. Storage tests of this product indicate that the IL-2 is stable in this form for more than three months at 2°C to 8°C.

The lyophilized mixture may be reconstituted by injecting a conventional parenteral aqueous injection such as water for injection, Ringer's injection, dextrose injection, dextrose and salt injection, or the like, into the vial. The injection should be added against the side of the vial to avoid excess foaming. The amount of injection added to the vial will typically be in the range of 1 to 5 ml, preferably 1 to 2 ml.

The reconstituted formulation is suitable for parenteral administration to humans or other mammals to provide IL-2 therapy thereto. Such therapy is appropriate for a variety of immunomodulatory indications such as T cell mutagenesis, induction of cytotoxic T cells, augmentation of natural killer cell activity, induction of IFN-gamma, restoration or enhancement of cellular immunity (e.g., treatment of immune deficient conditions), and augmentation of cell mediated anti-tumor activity.

The following example further illustrates the invention.

Example

The recombinant IL-2 used in this example is des-ala IL-2$_{ser125}$. The amino acid sequence of this IL-2 differs from the amino acid sequence of native human IL-2 in that it lacks the initial alanine of the native molecule and the cysteine at position 125 has been changed to serine. Samples of *E. coli* that produce this IL-2 have been deposited by Cetus Corporation in the American Type Culture Collection, 12301 Parklawn Drive, Rockville, Maryland, USA, on September 26, 1983 under accession number 39452 and on March 6, 1984 under accession number 39626 under the provisions of the Budapest Treaty.

329 mg of an RP-HPLC purified oxidized IL-2 product (protein concentration 0.94 mg/ml) in 60% 2-propanol, 6% acetic acid was diluted tenfold into 50 mM sodium acetate, 1 mM ethylene diamine tetraacetic acid (EDTA), 0.1% sodium dodecyl sulfate (SDS) at pH 5.5.

The IL-2 solution was then concentrated using a 10 sq. ft. hollow fiber cartridge (nominal molecular weight cut-off 10,000 daltons) to a volume of 600 ml and then diafiltered for 3 volumes against 50 mM sodium acetate, 1 mM EDTA, 0.1% SDS at pH 5.5. The material was then further diafiltered against 10 mM sodium phosphate containing 5 µg SDS/ml until the residual SDS reached a value of 131 µg SDS/ml protein. Approximately 255 mg IL-2 at a concentration of 0.6 mg/ml were recovered (425 ml).

Only 222 mg were used for the formulation which was carried out as follows: 370 ml of the IL-2 solution (222 mg, 0.6 mg/ml) was diluted with 10 mM sodium phosphate, pH 7.5 and 20% mannitol such that the final composition was:

0.25 mg/ml IL-2 ⎫
⎬ in 10 mM sodium phosphate, pH 7.5
5% mannitol ⎭

The solution was then sterile filtered through a 0.2 micron filter, filled into sterile vials (1.2 ml fill volume) and lyophilized. The product was sealed under vacuum.

The thus produced formulation has been used clinically in humans and has been well tolerated at dosages up to 2 million units/m$^2$ when administered as a continuous intravenous infusion or up to 1 million units/kg when administered as an intravenous or intramuscular bolus. Suitable indications for use of the recombinant IL-2 include:

1) treatment of immunodeficiency states, acquired, inborn, or induced by chemotherapy, immunotherapy, or irradiation;

2) enhancement of cell-mediated immune responses in the therapy of viral, parasitic, bacterial, malignant, fungal, protozoal, or mycobacterial or other infectious diseases;

3) induction of enhanced immunologic response of cells *ex vivo* in the treatment of infectious, malignant, rheumatic, or autoimmune diseases;

4) treatment of rheumatoid or other inflammatory arthridites;

5) treatment of diseases of abnormal immune response such as multiple sclerosis, systemic lupus erythematosis, glomerulonephritis, or hepatitis;

6) regulation of hematopoietic tumors or pre-malignant or aplastic abnormalities of hematopoietic tissue;

7) use as an adjuvant in induction of cell-mediated or humoral response to naturally occurring, administered neutral, chemically synthesized or modified, or recombinantly engineered vaccines or other antigens administered for therapeutic purposes;

8) use as a mediator of neurotransmission or as a psychoactive therapeutic, as an enkephalin for therapeutic purpose, or as a modifier of central nervous system function;

9) in a topical application for the treatment of above-mentioned disease states;

10) in combination with cytotoxic chemotherapy or irradiation or surgery in the treatment of malignant or pre-malignant diseases in a direct therapeutic or adjuvant setting;

11) in combination with agents with direct antiviral, anti-fungal, anti-bacterial, or anti-protozoal activity or in combination with drug therapy for typical and atypical m. tuberculosis;

12) in combination with other immune-modulating drugs, lymphokines, e.g., IL-1, IL-3, CSF-1, *alpha*-interferons, and gamma-interferons) naturally occurring or inducible anti-cellular toxins or molecules which mediate lysis or stasis or malignant cells in the treatment of malignant, infectious, autoimmune, or rheumatic diseases; and

13) for prophylaxis against infectious diseases and especially

regulating cell growth in a patient or treating a patient for a viral disease or stimulating natural killer cell activity in a patient or treating a patient having an immunodeficiency state, cancer or leprosy.

Claims

1. A recombinant IL-2 composition suitable for reconstituting in a pharmaceutically acceptable aqueous vehicle for parenteral administration to a patient to provide IL-2 therapy characterized by a sterile lyophilized mixture of:

(a) a therapeutically effective amount of selectively oxidized microbially produced recombinant IL-2 that is substantially free of non-IL-2 protein;

(b) a pharmaceutically acceptable water soluble carrier that does not affect the stability of the oxidized microbially produced IL-2 adversely; and

(c) a sufficient amount of a surface active agent to ensure the water solubility of the oxidized, microbially produced recombinant IL-2.

2. A composition according to claim 1 characterized in that the oxidized microbially produced IL-2 includes less than about 5% by weight non-IL-2 protein and the surface active agent is sodium dodecyl sulfate (SDS) or sodium deoxycholate.

3. A composition according to claim 2 characterized in that the sodium dodecyl sulfate is present at 100 to 250 μg per mg of IL-2.

4. A composition according to any one of claims 1—3 characterized in that the selectively oxidized microbially produced recombinant IL-2 constitutes 0.02% to 3.85% by weight of the mixture.

5. A composition according to any one of claims 1—4 characterized in that the recombinant IL-2 is des-ala IL-2$_{ser125}$ and the water-soluble carrier is mannitol.

6. A pharmaceutical composition for providing IL-2 therapy to a patient characterized by a sterile solution of:

(a) a mixture according to any one of claims 1—5 dissolved in

(b) a pharmaceutically acceptable aqueous parenteral injection, said solution containing in the range of 0.01 mg to 2 mg of the selectively oxidized microbially produced recombinant IL-2 per ml.

7. A method of making a recombinant IL-2 composition suitable for reconstituting in a pharmaceutically acceptable aqueous vehicle for parenteral administration to a patient to provide IL-2 therapy, which method comprises preparing a sterile lyophilized mixture of:

(a) a therapeutically effective amount of selectively oxidized microbially produced recombinant IL-2 that is substantially free of non-IL-2 protein;

(b) a pharmaceutically acceptable water soluble carrier that does not affect the stability of the oxidized microbially produced recombinant IL-2 adversely; and

(c) a sufficient amount of a surface active agent to ensure the water solubility of the oxidized, microbially produced recombinant IL-2.

8. A method of making a medicament for use in regulating cell growth in a patient or treating a patient for a viral disease or stimulating natural killer cell activity in a patient or treating a patient having an immunodeficiency state, cancer or leprosy, which method comprises preparing a sterile lyophilized mixture of:

(a) a therapeutically effective amount of selectively oxidized microbially produced recombinant IL-2 that is substantially free of non-IL-2 protein;

(b) a pharmaceutically acceptable water soluble carrier that does not affect the stability of the oxidized microbially produced IL-2 adversely; and

(c) a sufficient amount of a surface active agent to ensure the water solubility of the oxidized, microbially produced recombinant IL-2.

**Patentansprüche**

1. Rekombinante IL-2-Zusammensetzung, die zur Rekonstitution in einem pharmazeutisch verträglichen, wäßrigen Träger zur parenteralen Verabfolgung an einen Patienten zur Therapie mit IL-2 geeignet ist, gekennzeichnet durch ein steriles, gefriergetrocknetes Gemisch aus:

(a) einer therapeutisch wirksamen Menge selektiv oxidiertem, mikrobiologisch hergestelltem, rekombinantem IL-2, das im wesentlichen kein Nicht-Il-2-Protein enthält;

(b) einem pharmazeutisch verträglichen, wasserlöslichen Träger, der die Stabilität des oxidierten, mikrobiologisch hergestellten IL-2 nicht ungünstig beeinträchtigt; und

(c) einer ausreichenden Menge eines oberflächenaktiven Agens zur Gewährleistung der Wasserlöslichkeit des oxidierten, mikrobiologisch hergestellten, rekombinanten IL-2.

2. Zusammensetzung nach Anspruch 1, dadurch gekennzeichnet, daß das oxidierte, mikrobiologisch hergestellte IL-2 weniger als etwa 5 Gew.-% Nicht-IL-2-Protein enthält und das oberflächenaktive Agens Natriumdodecylsulfat (SDS) oder Natriumdesoxycholat ist.

3. Zusammensetzung nach Anspruch 2, dadurch gekennzeichnet, daß das Natriumdodecylsulfat in einer Menge von 100 bis 250 μg/mg IL-2 vorliegt.

4. Zusammensetzung nach einem der Ansprüche 1 bis 3, dadurch gekennzeichnet, daß das selektiv oxidierte, mikrobiologisch hergestellte, rekombinante IL-2 0,02 bis 3,85 Gew.-% des Gemisches ausmacht.

5. Zusammensetzung nach einem der Ansprüche 1 bis 4, dadurch gekennzeichnet, daß das rekombinante IL-2 des-Ala-IL-2$_{ser125}$ und der wasserlösliche Träger Mannit ist.

6. Pharmazeutische Zusammensetzung zur IL-2-Therapie eines Patienten, gekennzeichnet durch eine sterile Lösung von:

(a) einem Gemisch nach einem der Ansprüche 1 bis 5, gelöst in

(b) einer pharmazeutisch verträglichen, wäßrigen, parenteralen Injektion, wobei die Lösung 0,01 mg bis 2 mg des selektiv oxidierten, mikrobiologisch hergestellten, rekombinanten IL-2 pro ml enthält.

7. Verfahren zur Herstellung einer rekombinanten IL-2-Zusammensetzung, die zur Rekonstitution in einem pharmazeutisch verträglichen, wäßrigen Träger zur parenteralen Verabfolgung an einen Patienten zur Therapie mit IL-2 geeignet ist, bei dem man ein steriles, gefriergetrocknetes Gemisch herstellt aus:

(a) einer therapeutisch wirksamen Menge selektiv oxidiertem, mikrobiologisch hergestelltem, rekombinantem IL-2, das im wesentlichen kein Nicht-Il-2-Protein enthält;

(b) einem pharmazeutisch verträglichen, wasserlöslichen Träger, der die Stabilität des oxidierten, mikrobiologisch hergestellten, rekombinanten IL-2 nicht nachteilig beeinträchtigt; und

(c) einer ausreichenden Menge eines oberflächenaktiven Agens zur Gewährleistung der Wasserlöslichkeit des oxidierten, mikrobiologisch hergestellten, rekombinanten IL-2.

8. Verfahren zur Herstellung eines Medikaments zur Verwendung bei der Regulierung des Zellwachstums in einem Patienten oder bei der Behandlung eines Patienten wegen einer Viruserkrankung oder zur Stimulierung der natürlichen Killerzellen-Aktivität in einem Patienten oder zur Behandlung eines Patienten mit einer Immundefizienz, mit Krebs oder Lepra, bei dem man ein steriles, gefriergetrocknetes Gemisch herstellt aus:

(a) einer therapeutisch wirksamen Menge selektiv oxidiertem, mikrobiologisch hergestelltem, rekombinantem IL-2, das im wesentlichen kein Nicht-II-2-Protein enthält;

(b) einem pharmazeutisch verträglichen, wasserlöslichen Träger, der die Stabilität des oxidierten, mikrobiologisch hergestellten, rekombinanten IL-2 nicht nachteilig beeinträchtigt; und

(c) einer ausreichenden Menge eines oberflächenaktiven Agens zur Gewährleistung der Wasserlöslichkeit des oxidierten, mikrobiologisch hergestellten, rekombinanten IL-2.

**Revendications**

1. Composition d'IL-2 recombinante appropriée à la reconstitution dans un véhicule aqueux pharmaceutiquement acceptable pour administration parentérale à un patient pour fournir un traitement par IL-2, caractérisée par un mélange lyophilisé stérile de:

(a) une quantité thérapeutiquement efficace d'IL-2 recombinante produite par voie microbienne, sélectivement oxydée, qui est pratiquement exempte de protéine non IL-2;

(b) un véhicule soluble dans l'eau, pharmaceutiquement acceptable, qui ne nuit pas à la stabilité de l'IL-2 oxydée, produite par voie microbienne; et

(c) une quantité suffisante d'un surfactif pour garantir la solubilité dans l'eau de l'IL-2 recombinante oxydée, produite par voie microbienne.

2. Composition selon la revendication 1, caractérisée en ce que l'IL-2 oxydée, produite par voie microbienne, comprend moins d'environ 5% en poids de protéine non IL-2 et le surfactif est le dodécylsulfate de sodium (SDS) ou le désoxycholate de sodium.

3. Composition selon la revendication 2, caractérisé en ce que le dodécylsulfate de sodium est présent à raison de 100 à 250 μg par mg d'IL-2.

4. Composition selon l'une quelconque des revendications 1 à 3, caractérisée en ce que l'IL-2 recombinante produite par voie microbienne, sélectivement oxydée, constitue de 0,02 à 3,85% en poids du mélange.

5. Composition selon l'une quelconque des revendications 1 à 4, caractérisée en ce que l'IL-2 recombinante est la des-ala IL-2$_{ser125}$ et le véhicule soluble dans l'eau est le mannitol.

6. Composition pharmaceutique pour fournir d'un traitement par IL-2 à un patient, caractérisé par une solution stérile de

(a) un mélange selon l'une quelconque des revendications 1 à 5, dissous dans

(b) une solution aqueuse injectable par voie parentérale, pharmaceutiquement acceptable, ladite solution contenant de 0,01 à 2 mg de l'IL-2 recombinante produite par voie microbienne, sélectivement oxydée, par ml.

7. Procédé de préparation d'une composition d'IL-2 recombinante appropriée à la reconstitution dans une véhicule aqueux pharmaceutiquement acceptable pour administration parentérale à une patient pour fournir un traitement par IL-2, lequel procédé comprend la préparation d'un mélange lyophilisé stérile de

(a) une quantité thérapeutiquement efficace d'IL-2 recombinante produite par voie microbienne, sélectivement oxydée, qui est pratiquement exempte de protéine non IL-2;

(b) un véhicule soluble dans l'eau, pharmaceutiquement acceptable, qui ne nuit pas à la stabilité de l'IL-2 recombinante oxydée, produite par voie microbienne; et

(c) une quantité suffisante d'un surfactif pour garantir la solubilité dans l'eau de l'IL-2 recombinante oxydée, produite par voie microbienne.

8. Procédé de préparation d'un médicament pour utilisation dans la régulation de la croissance cellulaire chez un patient ou le traitement d'un patient pour une maladie virale, ou la stimulation de l'activité naturelle des cellules tueuses chez un patient, ou le traitement d'un patient souffrant d'un état immunodéficient, de cancer ou lèpre, lequel procédé comprend la préparation d'un mélange lyophilisé stérile de:

(a) une quantité thérapeutiquement efficace d'IL-2 recombinante produite par voie microbienne, sélectivement oxydée, qui est pratiquement exempte de protéine non IL-2;

(b) un véhicule soluble dans l'eau, pharmaceutiquement acceptable, qui ne nuit pas à la stabilité de l'IL-2 recombinante oxydée, produite par voie microbienne; et

(c) une quantité suffisante d'un surfactif pour garantir la solubilité dans l'eau de l'IL-2 recombinante oxydée, produite par voie microbienne.

## FIG. I

| FERMENTATION | |
|---|---|
| 4M UREA BATCH EXTRACTION | |
| 2-BUTANOL EXTRACTION | |
| S-200 COLUMN | 1% SDS, 50mM ACETATE, 2mM DTT, 1mM EDTA, pH 5.5 |
| G-25 COLUMN | 0.1% SDS, 50mM ACETATE, 1mM EDTA, pH 5.5 |
| OXIDATION | IODOSOBENZOIC ACID |
| G-25 COLUMN | 0.1% SDS, 50mM ACETATE, 1mM EDTA, pH 5.5 |
| RP-HPLC | ACETIC ACID, 2-PROPANOL |
| DILUTION / DIAFILTRATION | 1% SDS. 50mM ACETATE, 1mM EDTA, pH 5.5 |
| S-200 COLUMN | 1% SDS, 50mM ACETATE, 1mM EDTA, pH 5.5 |
| ULTRAFILTRATION | 2mM SODIUM PHOSPHATE, pH 7.5 |
| FORMULATION | |
| LYOPHILIZATION | |